# EUROPEAN PATENT APPLICATION

(11) **EP 4 834 346 A1**
(43) Date of publication of application: **04.11.2026**
(21) Application number: 25174061.9
(22) Date of filing: 02.05.2025
(51) Int. Cl.: A61K 31/505, A61K 45/06, A61P 25/00, A61P 25/18, A61P 25/24

(54) **ETRAVIRINE FOR THE TREATMENT OF NEURODEVELOPMENTAL DISORDERS**

(71) Applicant: Purposeful, Ike, 11251 Athens (GR)
(72) Inventor: Drakakis, Georgios, Athens (GR); Chomenidis, Charalampos, Athens (GR); Tsiliki, Georgia, Athens (GR); Vlachou, Thaleia, Athens (GR)
(74) Representative: Hamm&Wittkopp Patentanwälte PartmbB

(57) **Abstract**

The present invention relates to the use of Etravirine and a pharmaceutical composition containing Etravirine, preferably a tablet, for the treatment of neurodevelopmental disorders, in particular Autism Spectrum Disorder (ASD) and Attention-Deficit/Hyperactivity Disorder (ADHD).

## Description

### Technical Field of the Invention

The present invention relates to Etravirine and a pharmaceutical composition containing this drug for use in the treatment of neurological and developmental disorders, specifically for the treatment of Autism Spectrum Disorder (ASD) and Attention-Deficit/Hyperactivity Disorder (ADHD). In particular, the invention concerns pharmaceutical compositions comprising Etravirine for targeting the underlying neurobiological dysfunctions associated with ASD and ADHD.

### Background to the Invention

Etravirine is an antiretroviral agent used in the treatment of HIV-infection. Its mechanism of action involves direct inhibition of the HIV-1 reverse transcriptase enzyme. Etravirine has a terminal elimination half-life of 41 hours (Havens, 2020) and the adult dosage is 400 mg (200 mg twice daily or 400 mg once daily). It is approved for antiretroviral treatment-experienced children and adolescents aged 2-18 years old and weighing a minimum of 10 kg. It is commonly used as a second-line treatment due to its higher genetic barrier to resistance development. Etravirine is considered as safe and well tolerated for life long administration (Allavena *et al.,* 2016). The drug is extensively metabolized in the liver via cytochrome P450 enzymes, particularly CYP3A4, CYP2C9, and CYP2C19. The metabolites formed retain less than 90% of Etravirine's activity (Scholler-Gyure *et al.,* 2009).

The inventors have observed that Etravirine exhibits inhibitory activity against a wide range of neurodevelopment-associated kinases *in vitro.* Etravirine's ability to modulate these kinases is particularly relevant in the context of autism spectrum disorder (ASD) as well as in Attention-Deficit/Hyperactivity Disorder (ADHD), where dysregulated kinase signaling and synaptic dysfunction are implicated in both pathophysiologies.

Often diagnosed within the first three years of life, Autism Spectrum Disorder (ASD) is a genetically diverse group of neurobehavioral conditions marked by deficits in two key areas: social communication and interaction, and repetitive, stereotyped behaviors. Despite its increasing prevalence, the precise molecular mechanisms underlying ASD remain poorly understood. Core autistic behavioural features include deficits in social communication and restricted, repetitive sensory-motor behaviours. More precisely, individuals with ASD are characterized by hand-flapping and biting, poor eye contact, social anxiety, communication deficits and tactile hypersensitivity (Nisar *et al.,* 2019; Lord *et al.,* 2018).

The genetic basis of ASDs is highly heterogeneous, as hundreds of different genes have been implicated in their cause. Interestingly, most of the genes show expression profiles at the stage of early development, and their functionalities share strong enrichment in cell adhesion and mobility, cytoskeleton regulation, synapse formation and kinase signalling (Pinto, 2010; Gilbert, 2017). The spectrum of ASD risk genes reflects the disorder's complex molecular underpinnings, including roles in synaptic architecture, neurodevelopmental timing, and intracellular signaling (Gilbert, 2017).

The genetic abnormalities linked to ASD can be classified into three categories: (1) single gene mutations, which account for at least 5% of cases and involve genes such as SHANK3, FMR1, or MECP2, (2) copy number variants (CNVs) responsible for approximately 10% of cases, which include chromosomal alterations such as duplications, large deletions, inversions, and translocations, and (3) polygenic risk factors, where the combined effect of multiple common variants contributes to the overall risk (Varghese *et al.,* 2017).

Pharmacological therapies for ASD primarily target associated symptoms rather than the core features of the condition. To date, risperidone and aripiprazole remain the primary pharmacological options for addressing irritability and aggression in children with ASD. Other medications, such as Stimulants (e.g., methylphenidate) for hyperactivity, Selective Serotonin Reuptake Inhibitors (SSRIs) for anxiety and repetitive behaviors, and Melatonin for sleep disturbances, are prescribed off-label. Methylphenidate is also used for ADHD-like symptoms, but has a higher risk of side effects in ASD. Many of the pharmacological treatments mentioned above, have limited efficacy or significant side effects and evidence for managing co-occurring psychiatric conditions remains insufficient. As such, a major therapeutic gap exists in ASD treatment, given the lack of medications that directly address its core features. While some drugs can help manage associated symptoms such as irritability, hyperactivity, and sleep disturbances, they do not address the condition's underlying characteristics (Leclerc & Easley, 2015; Davico *et al.,* 2023).

To investigate potential treatments, ASD-related behaviors have been extensively studied using preclinical models, with the BTBR mouse strain being widely recognized for its strong face validity in mimicking core ASD traits observed in humans.

The behavioral deficits of ASD, as described above, are widely modelled in adult BTBR mice, which exhibit a wide spectrum of behavioral phenotypes, caused by a pool of genetic and epigenetic aberrations. As ASD is mainly caused by a combination of hundreds of *de novo* genetic mutations, BTBR mouse serves as a solid model for idiopathic ASD (Meyza & Blanchard, 2017).

Although the BTBR T⁺Itpr3^{tf}/J mouse was originally bred for studies on insulin-resistance, diabetes-induced nephropathy and phenyloketonuria, it has been widely used for studying ASD during the last decade, as displaying strong and consistent autism-related behaviors (McFarlane *et al.,* 2008). These behaviors are: social behavioral deficits, reduced vocalizations, highly repetitive behavior and impaired cognitive behavior (Zerbi *et al.,* 2021). More specifically, BTBR mice show reduced interest in engaging with conspecifics and decreased social interaction overall. When placed in a social test, such as the three-chamber test, they exhibit a reduced preference for social stimuli, which resembles social communication deficits seen in ASD. These mice, also, engage in excessive grooming and other stereotypical motor activities, such as repetitive sniffing and head weaving. These repetitive behaviors mirror the restricted and repetitive behaviors observed in humans with ASD. Additionally, BTBR mice show a reduced capacity for ultrasonic vocalizations, a form of communication commonly used by mice in social contexts. This deficit in communication recapitulates the social communication difficulties found in individuals with ASD, particularly individuals with severe forms of disorder. These mice also exhibit sensory processing issues, such as hyperreactivity to auditory stimuli, which are also characteristics of individuals with ASD, who may have atypical responses to sensory input (Viscomi *et al.,* 2025).

Regarding the genotype of BTBR mice, it has been found that they carry autism-related traits in chromosomes 1,3,4,6,9,10,12 and X chromosome (Viscomi *et al.,* 2025). Transcriptomic analysis in BTBR brain has also revealed dysregulated genes in important brain areas for behavior ( hippocampus, cortex and cerebellum) such as CDK5 (Li *et al.,* 2022) and GSK3 (De Groot *et al.,* 2017), which are also involved in ASD and are analysed extensively later. These cause distinct neuroanatomical and functional features that align with the behavioral phenotypes observed in these mice, such as agenesis of corpus callosum, alterations in the morphology of brain ventricles and amygdala structures, as well as decreased neurogenesis (Endo *et al.,* 2025). Altered neural connectivity, synaptic dysfunction, and neuroinflammation also play an important role in the model's phenotype (Mutovina *et al.,* 2022).

Several drugs targeting neurobiological pathways believed to be disrupted in ASD have been tested in the BTBR model. For instance, treatments that modulate the serotonergic system (Amodeo *et al.,* 2016), neuroinflammation (Bakheet *et al.,* 2016) and defective neurotransmission (Amodeo *et al.,* 2014) have been evaluated for their ability to alleviate social and repetitive behaviors. Regulating the mTOR signaling pathway can also reverse or mitigate behavioral deficits in BTBR mice (Burket *et al.,* 2014). By using this model, researchers can explore the biological mechanisms underlying autism, test additional potential pharmacological and behavioral treatments, and develop strategies for improving outcomes in individuals with ASD.

Building on its impact in ASD models, Etravirine has also exhibited beneficial effects in ADHD-related contexts, pointing to its wider therapeutic potential.

Attention-Deficit/Hyperactivity Disorder (ADHD) is a neurodevelopmental disorder characterized by persistent impairments in attention regulation, impulse control, and hyperactivity, which significantly impact an individual's daily life and activities (Polanczyk *et al.,* 2007). ADHD is primarily a genetically driven disorder, with heritability estimates ranging between 77% and 80%. Polygenic risk factors contribute to the disorder, with identified genetic loci associated with dopaminergic neurotransmission, including DRD4, DAT1, and COMT, suggesting dysregulation of catecholaminergic pathways in the prefrontal cortex and striatum. While genetic predisposition is a dominant factor, environmental influences such as prenatal exposure to toxins (e.g., nicotine, alcohol), low birth weight, and perinatal complications also modulate the disease's expression and severity (Balogh *et al.,* 2022).

Dopamine is found to be dysregulated in individuals with ADHD resulting in difficulties sustaining attention, impulsivity, and an increased need for external stimulation. Variations in the DRD4 gene lead to reduced receptor efficiency, weakening dopamine signaling in areas responsible for cognitive control. Similarly, polymorphisms in the DAT1 (SLC6A3) gene affect dopamine transporter activity by increasing dopamine reuptake and thereby lowering its availability in the synapse. Additionally, mutations in the DBH gene, which regulates dopamine-to-norepinephrine conversion, may further disrupt neurotransmitter balance, contributing to executive dysfunction. These findings support the theory that ADHD is driven, at least in part, by impaired dopamine regulation, which affects reward processing and behavioral control. This aligns with the effectiveness of stimulant medications like methylphenidate, which work by increasing dopamine levels in the brain (Blum *et al.,* 2008).

ADHD is classified into three primary clinical subtypes: predominantly inattentive, predominantly hyperactive-impulsive, and a combined type of these two (Drechsler *et al.,* 2020). The disorder exhibits a male predominance (approximately 2:1 in children), though females often present more subtle symptoms, leading to underdiagnosis. The global prevalence is estimated at 5-7% in children and 2-5% in adults, with around 30-60% of cases continuing into adulthood. ADHD is frequently comorbid with psychiatric conditions, such as anxiety, mood disorders, learning disabilities, and Autism Spectrum Disorder (ASD), complicating diagnosis and management (Balogh *et al.,* 2022). ADHD is diagnosed based on standardized criteria outlined in the DSM-5 and ICD-11 classifications, requiring symptoms to persist for at least six months across multiple settings, accompanied by clear evidence of functional impairment. Due to its heterogeneous nature, a comprehensive assessment of executive function deficits, cognitive processing speed, and emotional dysregulation is essential for an accurate diagnosis (Drechsler *et al.,* 2020).

Therapeutic intervention for ADHD follows a multimodal approach, incorporating pharmacological, behavioral, and environmental modifications. First-line pharmacotherapy includes stimulant medications (e.g., methylphenidate, amphetamine derivatives), which enhance dopamine and norepinephrine neurotransmission, leading to improved cognitive function and behavioral control. Non-stimulant alternatives, such as atomoxetine (a selective norepinephrine reuptake inhibitor) and α-2 adrenergic agonists (guanfacine, clonidine), offer additional therapeutic options. Behavioral therapy, including Cognitive Behavioral Therapy (CBT), executive function training, and parent management programs, serves as an adjunct to pharmacotherapy, particularly in pediatric populations. Despite the availability of pharmacological treatments for ADHD, a significant gap in effective solutions remains. Stimulant medications are effective for many individuals, but are not well-tolerated and can cause adverse effects, including insomnia, decreased appetite, and increased anxiety. While non-stimulant alternatives offer additional treatment options, they may be less effective. Furthermore, ADHD in adults is often underdiagnosed and inadequately treated (Mechler *et al.,* 2022).

To further explore the neurobiological underpinnings of ADHD and potential therapeutic interventions, the DAT knockout (KO) mouse model is widely used, as it exhibits hyperactivity and other hallmark features of the disorder.

ADHD is often associated with abnormalities in the dopamine system, specifically the dopamine transporter (SLC6A3, DAT), which is responsible for reuptaking dopamine into presynaptic neurons (Brown *et al.,* 2010). Dopamine systems have been associated with the regulation of both baseline and stimulant-induced movement. The dopamine transporter plays a crucial role in dopamine compartmentalization, controlling the dynamic changes in intracellular, synaptic, and extrasynaptic dopamine concentrations (Hall *et al.,* 2014). In the DAT-KO mouse, the gene for this dopamine transporter is deleted, leading to an increase in extracellular dopamine levels (Bossé *et al.,* 1997). DAT knockout (DAT-KO) mouse model is considered a good model for Attention-Deficit/Hyperactivity Disorder (ADHD), as it closely mimics several key features of the disorder, particularly those related to dopamine dysregulation, which is thought to play a central role in ADHD (Viggiano *et al.,* 2003).

The absence of DAT leads to several notable behavioral and physiological changes in the DAT-KO mouse. One of the most significant alterations is abnormal motor activity, such as hyperactivity or abnormal locomotion patterns, which mimic a hallmark of ADHD (Cyr *et al.,* 2003). The increased activity levels in the model often coexist with impulsivity, a key symptom of ADHD. In addition, DAT-KO mice show impairments in cognitive tasks, particularly those requiring attention, decision-making, and inhibitory control, which closely resemble the attentional deficits observed in ADHD patients. These mice also exhibit alterations in reward processing, leading to abnormal responses to rewarding stimuli and impaired reinforcement learning. Additionally, the DAT-KO mouse model shows changes in circadian rhythms, social behavior, and anxiety-like responses, further demonstrating the broad impact of dopamine signaling on both cognitive and emotional functioning (Efimova *et al.,* 2016).

Various treatments have been investigated to evaluate their potential in alleviating symptoms in DAT-KO mice. Pharmacological approaches include the administration of dopaminergic agents, such as dopamine receptor agonists or antagonists (psychostimulants), to correct or compensate for the altered dopamine signaling (Del'Guidice *et al.,* 2014). Additionally, reuptake inhibitors targeting other neurotransmitter systems, like serotonin, have been tested to determine if they can modulate the hyperdopaminergic state (Hall *et al.,* 2014). As the DAT-KO mouse model shares several of the core symptoms and underlying neurobiological mechanisms of ADHD, it serves as a valuable preclinical model for testing potential treatments and exploring therapeutic strategies.

Although ASD and ADHD are distinct clinical entities, they exhibit substantial overlap in behavioral, neurobiological, and genetic domains-suggesting shared underlying mechanisms. Epidemiological studies indicate that 50-70% of individuals with ASD display ADHD symptoms, while 20-50% of those diagnosed with ADHD meet criteria for ASD. This comorbidity reflects common disruptions in prefrontal-striatal circuitry, impaired executive functioning, and dysregulated dopaminergic and serotonergic signaling. Both conditions are also marked by deficits in cognitive flexibility, atypical sensory processing, and challenges in social reciprocity (Hours *et al.,* 2022).

At a molecular level, convergent genetic pathways further reinforce this overlap. For example, KDM6B, a histone demethylase crucial for chromatin remodeling and neurodevelopment, has been implicated in both disorders through its association with ADHD-linked genomic regions. Other shared genes-such as CAPRIN1 and CELSR3-play key roles in synaptic function, neuronal differentiation, and circuit formation, directly linking genetic variation to cognitive and behavioral regulation across both ASD and ADHD (Zhou *et al.,* 2022).

### Detailed Description of the Invention

The objective underlying the present invention was the provision of a remedy for the treatment of neurodevelopmental disorders. This objective has been solved by the subject matter as defined in the claims.

Embodiments of the invention are described below, by way of example only with reference to and as illustrated in the following figures:

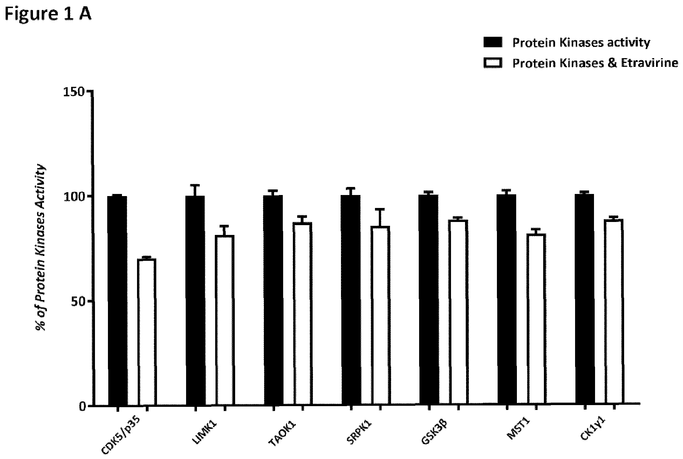

**Figure 1A** depicts that Etravirine inhibits kinases implicated in the pathogenesis of neurodegenerative diseases. Kinase inhibition was measured *in vitro* using a radiometric assay that quantifies the incorporation of radiolabeled phosphate into substrate peptides. Recombinant kinases were incubated with Etravirine, and the resulting phosphorylation activity was assessed to determine the compound's inhibitory effects on individual kinases. Fig. 1A is a bar plot which demonstrates Kinases implicated in neurodegenerative diseases expression levels before and after Etravirine treatment in an *in vitro* protein kinase activity assay, normalized to the baseline expression of each kinase prior to treatment. The neuro-related kinases significantly inhibited by Etravirine are: Cyclin-Dependent Kinase 5 (CDK5), LIM Kinase 1 (LIMK1), Human Serine/threonine-protein kinase TAO1 (TAOK1), Serine/Arginine-Rich Protein-Specific Kinase 1 (SRPK1), Glycogen Synthase Kinase 3 Beta (GSK3β), Mammalian Ste20-like kinase 1 (MST1) and Casein Kinase 1 Gamma 1 (CK1γ1).

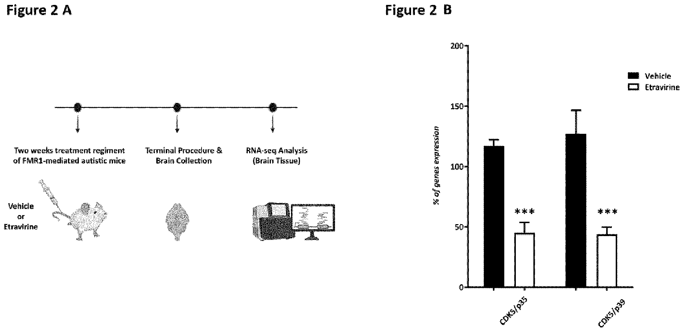

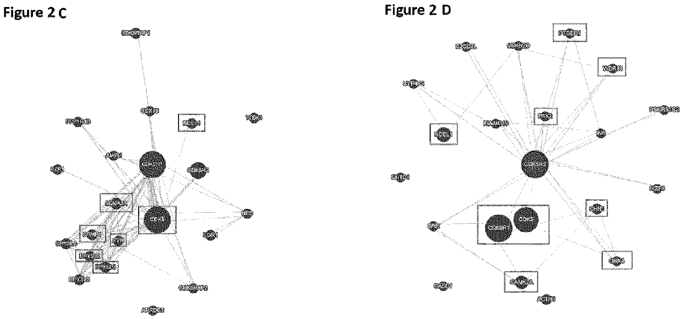

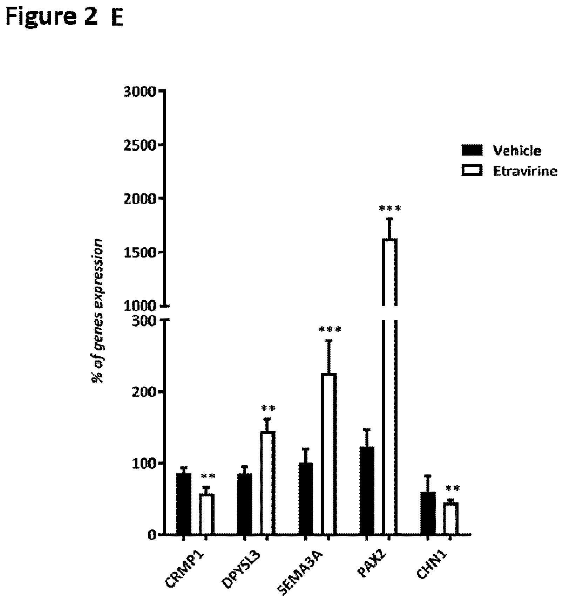

**Figure 2** illustrates the *in vivo* relevance of Etravirine's molecular activity in a validated autistic mouse model. Whole-brain RNA sequencing was performed in FMR1-knockout mice, a widely used model of Autism Spectrum Disorder (ASD), to assess transcriptomic alterations. In particular, Fig. 2A is a schematic representation of the *in vivo* experiment conducted by the inventors. Fig. 2B is a Bar graph showing CDK5R1 (CDK5 p35) and CDK5R2 (CDK5 p39) relative brain mRNA expression levels of Vehicle-treated ASD mouse model compared to Etravirine-treated ASD mouse model. Fig. 2C is a schematic representation of the CDK5R1 Gene Interaction Network. Genes outlined in red boxes are known to be associated with ASD. Fig. 2D is a schematic representation of the CDK5R2 Gene Interaction Network. Genes outlined in red boxes are known to be associated with ASD. Fig. 2E is a Bar graph showing CRMP1, DPYSL3, SEMA3A, PAX 2 and CHN1 relative brain mRNA expression levels of Vehicle-treated ASD mouse model compared to Etravirine-treated ASD mouse model, indicating a significant effect of Etravirine on molecular pathways involved in ASD pathogenesis.

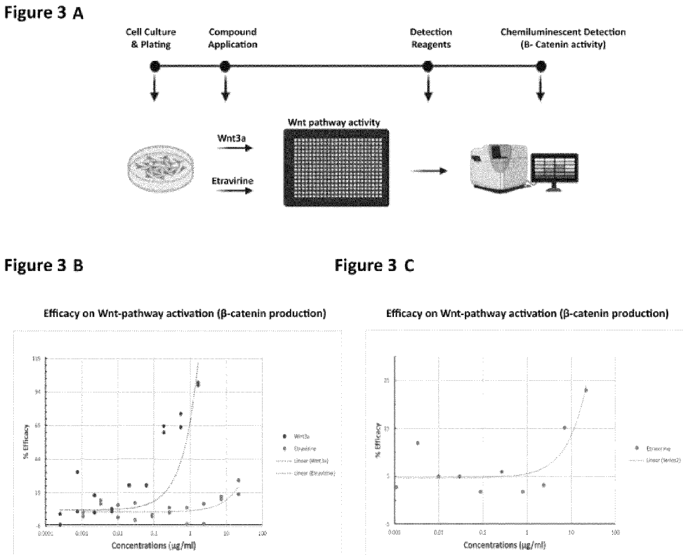

**Figure 3** depicts that Etravirine has the ability to normalise Wnt Pathway. Specifically, Fig. 3A is a schematic representation of the *in vitro* experiment conducted by the inventors. Fig. 3B is a graph representing β-catenin fluorescence activity in response to Wnt3a activator and Etravirine treatment, illustrating their respective effects on Wnt signaling activity. Fig. 3C is a graph representing β-catenin fluorescence activity in response to Etravirine treatment, illustrating its respective effects on Wnt signaling activity.

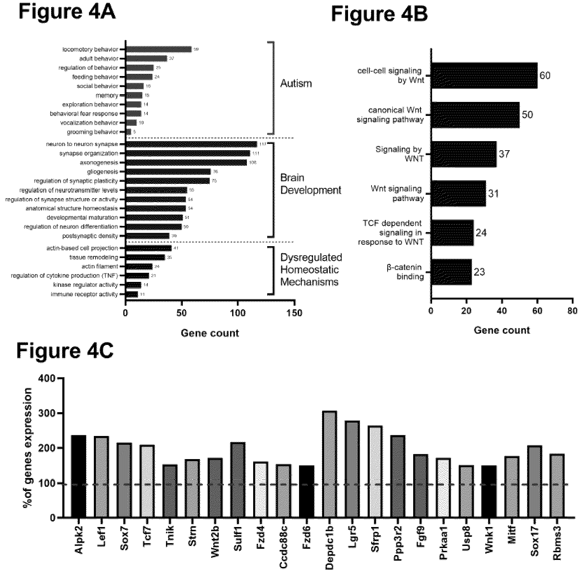

**Figure 4** illustrates the *in vivo* molecular pathways affected by Etravirine treatment in FMR1-knockout mice, based on the whole-brain RNA sequencing data presented in Figure 2A. Fig. 4A highlights the implication of specific pathways in the reversal of autistic-like behaviors following Etravirine administration, such as autism-related and brain development-related pathways, as well as pathways regulating systemic homeostasis (Dysregulated Homeostatic Mechanisms). Fig. 4B focuses on Wnt signaling pathways modulated by Etravirine. Fig. 4C displays genes involved in Wnt signaling that are upregulated as a result of the treatment.

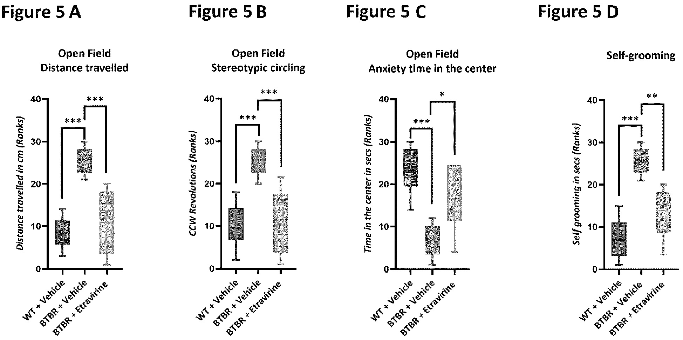

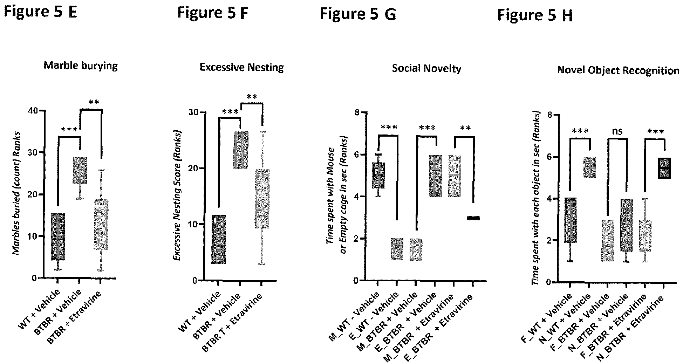

**Figure 5** illustrates the therapeutic efficacy of Etravirine (at a daily dose of 80 mg/kg for at least 14 days) to ameliorate ASD-like symptoms in BTBR mice and restore them to wild-type levels (all mice were males at their 2 month-old age and in C57BL/6J background). These phenotypes - including hyperactivity, stereotypy, cognitive impairment, antisocial behavior, impaired memory and learning - highly resemble core traits of ASD. The amelioration of the phenotypes are depicted in Kruskal-Wallis graphs (Fig. 5A, 5B, 5C, 5D, 5E, 5F) and Friedman graphs (Fig. 5G and 5H) showing pairwise comparisons for multiple behavioral tests performed in WT mice treated with Vehicle (n=10) and BTBR mice treated either with Vehicle (n=10) or Etravirine (n=10). Graphs Fig. 5A, 5B, 5C, 5D, 5E and 5F present pairwise comparisons to the BTBR Vehicle mice, and Fig. 5E and 5H compare for each of the conditions: empty cell versus non-empty cell (Fig. 5G) and familiar versus novel mouse (Fig. 5H). Statistically significant comparisons are indicated by *** (p-value≤0.001), ** (p-value≤0.01) or * (p-value≤0.05), whereas non- significant comparisons are indicated by ns.
   More specifically, Fig. 5A, 5B and 5C demonstrate the improvement in hyperactivity, stereotypic circling behaviours and anxiety-related behaviors exhibited by BTBR mice upon Etravirine treatment. These were evaluated in an Open field test (a structure surrounded by walls to prevent mouse escape), where BTBR mice exhibited increased total distance traveled in a given time (Fig. 5A; assessment of anxiety and hyperactivity), elevated circling (Fig. 5B; assessment of stereotypy) and decreased time spent in the center of the Open field (Fig. 5C; assessment of anxiety). These alterations were significantly improved following Etravirine treatment. Fig. 5D, 5E and 5F illustrate the efficacy of Etravirine to ameliorate repetitive, stereotyped and compulsive behaviours of BTBR mice, which are exhibited as extensive self-grooming (Fig. 5D), marble-burying (Fig. 5E) and nesting (Fig. 5F). Impaired fine motor skills control, which is also indicated by extensive nesting (Fig. 5F), is ameliorated upon Etravirine treatment as well. Impaired learning, recognition memory and social interaction of BTBR mice, tested by the Three-Chamber Sociability Test, were also ameliorated (Fig. 5G and 5H). Treatment with Etravirine reverses the difficulty of BTBR mice to distinguish between a novel (N) and a familiar (F) mouse in the Novel Object Recognition test (Fig. 5H), which indicates impaired learning and deficits in recognition memory , and also improves the impaired social interaction of BTBR mice, which, when untreated, tend to spend more time in an empty cage (E), rather than in a cage with another mouse present (M) (Fig. 5G).

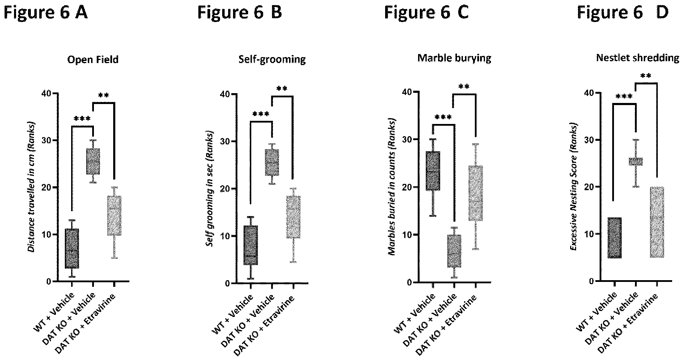

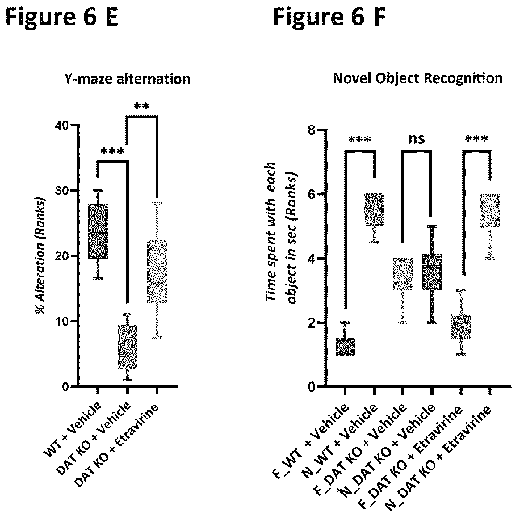

**Figure 6** displays the therapeutic efficacy of Etravirine (at a daily dose of 80 mg/kg for at least 14 days) to ameliorate ADHD-like symptoms in DAT-KO mice and restore them to wild-type levels (all mice were males at their 2 month-old age and in C57BL/6J background). These symptoms include hyperactivity, stereotypy, cognitive impairment, compulsivity and impaired memory. The amelioration of the phenotypes are depicted in Kruskal-Wallis graphs (Fig. 6A, 6B, 6C, 6D and 6E) and Frieman graph (Fig. 6F) showing multiple behavioral tests performed in WT mice treated with Vehicle (n=10) and DAT-KO mice treated either with Vehicle (n=10) or Etravirine (n=10). Fig. 6A, 6B, 6C, 6D and 6E present pairwise comparisons to the DAT-KO Vehicle mice, and Fig. 6E compares for each of the conditions familiar versus a novel mouse. Statistically significant comparisons are indicated by *** (p-value≤0.001), ** (p -value≤0.01) or * (p-value≤0.05), whereas non- significant comparisons are indicated by ns.

More specifically, Fig. 6A illustrates the improvement of hyperactivity levels in DAT-KO mice upon Etravirine treatment, which was studied by an Open field test, where DAT-KO mice exhibited increased total distance traveled in a given time. Additionally, the decrease in self-grooming observed in DAT-KO mice upon Etravirine treatment (Fig. 6B) further reflects a reduction in hyperactive behavior. Interestingly, Etravirine administration improves deficient attention, which is indicated by the impaired marble burying by DAT-KO mice (Fig. 6C). Repetitive and compulsive-like behavior of DAT-KO mice, indicated by extensive nestlet shredding, is also ameliorated with Etravirine treatment (Fig. 6D). Impaired short-term memory processes, deficits in cognitive flexibility and spatial memory in DAT-KO mice were also improved following Etravirine treatment, as evidenced by increased spontaneous alternation in the Y - maze test (Fig. 6E). Lastly, Etravirine improves the ability of DAT-KO mice to distinguish between a novel (N) and a familiar (F) mouse in the Novel Object Recognition test (Fig. 6F), which indicates impaired learning and deficits in recognition memory .

BTBR T+Itpr3tf/J mice and DAT-KO (B6.129X1(FVB)-Slc6a3tm1Hhg/J) were purchased by Jackson Laboratory and kept in C57BL/6J background. They were housed in plastic cages (35 x 30 x 12 cm), at room temperature (21 ± 2 °C), relative humidity (55 ± 5%), a 12-hour light-dark cycle and air exchange (16 times per hour). They were fed and watered ad libitum.

Treatment: Etravirine was administered intraperitoneally at a dose of 80 mg/kg for at least 14 days; Vehicle had a concentration of DMSO (2 mg/mL) and then further diluted in PBS at PH 7.2. All behavioural tests were conducted during the light phase, once 14-day treatment was over. Experiments were conducted in line with UK legislation for animal use (Scientific Procedures, Act 1986). All experiments are conducted with the experimenter blind to genotype and drug treatment. Separate investigators prepared and coded dosing solutions, allocated the mice to the study treatment groups, administered the animals, and collected the behavioural data.

### Studies relating Etravirine to phenotypic effects in ASD and ADHD.

We have previously shown in the application PCT/US24/54523 filed May 11, 2024, expressly incorporated herein fully by reference, that Etravirine unexpectedly ameliorated the FXS phenotypes in FMR1 mice and could therefore be employed as a useful and efficacious treatment for Fragile X syndrome and FMR1-mediated autism.

The inventors have now conducted a comprehensive *in vitro* kinase assay to elucidate the molecular mechanisms underlying Etravirine's therapeutic effects and have revealed that Etravirine inhibits seven kinases which are strongly involved in neuronal development and synaptic plasticity. The neuro-related kinases most significantly inhibited by Etravirine include: Cyclin-Dependent Kinase 5 (CDK5), LIM Kinase 1 (LIMK1), Human Serine/threonine-protein kinase TAO1 (TAOK1), Serine/Arginine-Rich Protein-Specific Kinase 1 (SRPK1), Glycogen Synthase Kinase 3 Beta (GSK3β), Mammalian Ste20-like kinase 1 (MST1) and Casein Kinase 1 Gamma 1 (CK1γ1) (Figure 1A).

CDK5 is a proline directed serine/threonine kinase highly involved in neuronal migration, essential for the neurosynaptic plasticity and the cytoskeletal structure. CDK5 plays a crucial role in dendritic spine formation because of its aberrant neurotransmitter release which affects short-term memory and CDK5 dysregulation leads to cognitive deficits directly linked to Autism and ADHD Neurodevelopmental Disorders (Stamova *et al.,* 2013). Research studies on various environmental factors have shown that elements, such as lead exposure can induce Tau phosphorylation through the CDK5 pathway contributing to ASD neurological disorders (Gassowska *et al.,* 2016). Furthermore, reduced CDK5 activity is linked with alteration in DAT expression and dopamine uptake in ADHD animal models, suggesting a crucial role between CDK5's molecular action and dopamine signaling regulation in ADHD (Kim *et al.,* 2019).

LIMK1 is a kinase with an active role in actin cytoskeletal remodeling, vital for neuronal synaptic connectivity and plasticity. The genetic mutations affecting LIMK1 contribute to ASD-related phenotypes, such as deviant social behavior and executive dysfunction (Yao *et al.,* 2021). A study investigating the timing-dependent effects of fluoxetine (a known SSRI) on neurogenesis and behavior, highlighted the crucial involvement of LIMK1 in synaptic remodeling and demonstrated that its altered activity leads to phenotypic changes relevant to ASD and ADHD (Yao *et al.,* 2021). Similarly, LIMK1 involvement in ADHD is exhibited by its role in synaptic signal transduction and attention control (Zimmermann *et al.,* 2015).

Serine/Arginine-Rich Protein-Specific Kinase 1 (SRPK1) actively modulates alternative RNA splicing and diverse neuronal gene networks. Aberrant SRPK1 signaling is linked to ASD pathogenesis through its effects on synaptic transcriptomics (Stamova *et al.,* 2013). Moreover, spatiotemporal gene expression profile reveals that SRPK1 expression is involved in various neurodevelopmental and somatic events throughout the lifetime (Wang *et al.,* 2024).

Consistent with the kinases previously noted, TAO Kinase 1 is strongly implicated in neuronal development, including axon outgrowth and neuronal migration, and plays a pivotal role in hippocampal-dependent learning and synaptic plasticity. Dysfunctions in TAOK1 signaling contribute to ASD cognitive deficits and neurodevelopmental instability ( Wang *et al.,* 2023). TAOK1 is also studied in ADHD, in which dysregulation is prominently observed in attention-related signaling pathways (Byeon & Yadav, 2024).

Finally, Glycogen synthase kinase-3 beta (GSK-3β) has been implicated in various neurological disorders, including ASD and ADHD, due to its crucial role in cellular processes and neuronal function, making it a potential target for therapeutic interventions (Jones *et al.,* 2025). GSK3β is a crucial component of multiple neurodevelopmental signaling pathways, including Wnt/β-catenin and mTOR, both implicated in ASD (Jiang *et al.,* 2022). Notably, GSK3β inhibition has shown promise in ADHD by restoring dopamine-related synaptic function (Yen *et al.,* 2015) and extensive genomic analyses have identified it as one of the genes most strongly associated with neurodevelopmental disorders (Mines *et al.,* 2010).

MST1 kinase is a key component of the Hippo pathway which modulates apoptosis and neuronal differentiation, and its dysregulation is strongly implicated in neurodegenerative and neurodevelopmental disorders (Fu *et al.,* 2022).

Meanwhile, CK1γ1 plays a pivotal role in Wnt/β-catenin signaling and circadian rhythm regulation, both essential for neurodevelopment and cognitive function (Pande *et al.,* 2019). Notably, pharmacological inhibition of MST1 and CK1γ1 by Etravirine is proposed as a novel strategy for neurodevelopmental disorder therapy. By targeting MST1, Etravirine may promote neuronal resilience and plasticity, counteracting the apoptotic pathways linked to ASD and ADHD. Similarly, CK1γ1 inhibition could modulate Wnt signaling associated with these conditions, ultimately restoring cognitive and behavioral function.

The inventors demonstrate that *in vitro* inhibition of these neuro-related kinases, as analyzed above, along with the significant behavioral improvements observed in an FXS mouse model, support Etravirine's therapeutic potential in ASD and ADHD. Through its inhibitory effects, Etravirine may help restore synaptic structure, regulate splicing mechanisms, and re-establish synaptic homeostasis, collectively leading to an overall improvement in the ASD and ADHD phenotype.

Based on the above, the invention relates to composition for use in the treatment of neurological and developmental disorders, and in particular for a range of ASD and ADHD.

As used herein, the terms "treatment", "treating", "treat" and the like, refer to obtaining a desired pharmacological and/or physiologic effect. "Treatment" as used herein covers in particular the symptomatic treatment of a disease in a mammal, particularly in a human.

The composition is intended for the treatment of diseases and disorders associated with the significant dysregulation of the seven kinases inhibited by Etravirine (Figure 1A).

Disorders associated with disruptions in the molecular pathways regulated by these specific kinases can be categorized based on the individual kinase affected. Accordingly, patients exhibiting molecular dysregulation within signaling pathways modulated by these seven kinases may benefit from treatment with the pharmaceutical composition of the present invention comprising Etravirine.

**CDK5** dysregulation is highly correlated with Schizophrenia, Epilepsy, Major Depressive Disorder (MDD), Intellectual Disability (ID), Dyslexia, Dyscalculia, Speech and Language Disorders and Rett Syndrome.

Schizophrenia, a psychiatric disorder involving cognitive dysfunction, hallucinations, and impaired executive function, is, also, linked with CDK5 abnormal regulation through p53 down-regulated expression (Engmann *et al.,* 2011). Furthermore, *NAPB-*related epilepsy is a condition which demonstrates synaptic vesicle recycling effects due to CDK5 dysregulation (Long, 2024). Intellectual Disability marked by global developmental delays and cognitive impairments, exhibits severe mutations on the CDK5 gene (Moncini *et al.,* 2016). Major Depressive Disorder (MDD) is characterised as an affective disorder with persistent low mood, anhedonia, and cognitive impairment which is linked to CDK5-mediated phosphorylation of Sirt2 (Zhang *et al.,* 2018). Moreover, dyslexia and dyscalculia is indicative of learning disorders affecting reading and mathematical processing and its phenotype is highly associated with CDK5 gene (Burkhardt *et al.,* 2012). Finally, Rett syndrome is a neurodevelopmental disorder characterized by progressive motor dysfunction, loss of speech, and stereotypic hand movements. In this syndrome the key transcriptional methyl-CpG-binding protein 2 which regulates the physiological CDK5 function is mutated (Liang *et al.,* 2015).

**LIMK1** dysregulation is significantly correlated with Williams Syndrome and Schizophrenia. Williams Syndrome is a genetic disorder causing intellectual disability, hypersociability, and visuospatial deficits and is caused by deletions in the 7q11.23 region which affects the basal expression of the LIMKI1 gene (Long, 2024). Schizophrenia is also linked to LIMK1 gene, as its pharmacological inhibition restores synaptic plasticity and normal behavior in mice (Gory-Faure *et al.,* 2021).

**SRPK1** dysregulation is linked with Intellectual Disability (ID) as its functional altered expression leads to abnormal neurodevelopmental signaling (Bustos *et al.,* 2020).

**MST1** dysregulation is linked with Epilepsy-related neuro-inflammation. Epilepsy is considered as a severe neurological disorder characterized by abrupt abnormal electrical activity in the brain and recurring spontaneous seizures. The MST1 expression was significantly high both in patients at their peak of seizures, as well as in the temporal cortex and hippocampus of epileptic rat models (Xia *et al.,* 2025). Moreover, MST1 altered gene expression plays a critical role in induced apoptosis and neuro-inflammation in hippocampal neurons (Yang *et al.,* 2016).

**GSK3β** dysregulation is related to Phelan-McDermid Syndrome, Tuberous Sclerosis Complex (TSC), Intellectual Disability (ID) and Rett Syndrome. Phelan-McDermid Syndrome is observed as a neurodevelopmental disorder characterized by intellectual disability and ASD-like behaviors. Pharmacological GSK3β inhibitors, like lithium, have been investigated in clinical trials for Phelan-McDermid Syndrome patients, aiming to improve social communication deficits by stabilizing synaptic functions. These findings suggest a link between over-activation of GSK3β and phenotypic characteristics of the Phelan-McDermid Syndrome (Egger *et al.,* 2017). Tuberous Sclerosis Complex (TSC) is a genetic disorder characterized by benign tumors, epilepsy, and neurodevelopmental delay. TSC is characterized by mTORC1 hyperactivation due to *TSC1* or *TSC2* mutations. This leads to GSK3β inhibition (via S6K1 phosphorylation), disrupting glycogen metabolism and contributing to cellular growth dysregulation (Mak *et al.,* 2005). GSK3β dysregulation is, also, implicated in various neurodevelopmental disorders, including those leading to Intellectual Disability (ID). Finally, Rett syndrome, in addition to its association to CDK5, is also linked with GSK3β dysfunction. Specifically, GSK3β expression is dysregulated leading to increased NF-κB signaling and neuroinflammation, contributing to the disorder's neurodevelopmental symptoms (Jorge-Torres *et al.,* 2018).

The composition may be utilized for the treatment of diseases significantly impacted by the dysregulation of the kinases analyzed above.

To further explore the *in vivo* relevance of these findings, the inventors performed whole-brain RNA sequencing in a validated FMR1-knockout mouse model of Autism Spectrum Disorder (ASD). This model recapitulates core behavioral and molecular phenotypes of human ASD and provides a high-resolution transcriptomic profile of neurodevelopmental dysregulation. Remarkably, the RNA-seq analysis revealed that the CDK5's regulatory subunits CDK5/p35 and CDK5/p39 were considerably downregulated in the FMR1-KO brain (Figure 2), in complete accordance with the inhibitory pattern shown in the *in vitro* kinase panel assay. This *in vivo* evidence corroborates the essential role of these kinases in disease pathology and supports their inhibition as a mechanistic basis for the therapeutic efficacy of the present composition.

To investigate the downstream consequences of this kinase modulation, the inventors analyzed protein-protein interaction networks and transcriptional outputs related to ASD-associated genes (Figure 2). This analysis revealed that Etravirine significantly regulates multiple genes directly implicated in ASD pathophysiology, further underscoring its potential for disease modification. Specifically, CRMP1 (Collapsin Response Mediator Protein 1), a gene whose over-expression is linked to impaired neurite outgrowth and ASD-like phenotypes (Ramirez-Celis *et al.,* 2022), was found to be downregulated following Etravirine administration, suggesting therapeutic correction of aberrant expression levels. DPYSL3, a key modulator of axon guidance and neuronal differentiation, and a gene whose knockout results in ASD-relevant behavioral deficits in animal models (Tsutiya *et al.,* 2017), was upregulated upon treatment, indicating a potential enhancement of neurodevelopmental pathways. Furthermore, SEMA3A, a class 3 semaphorin crucial for dendritic architecture and synaptic organization, and known to be mutated in ASD (Matrone & Ferretti, 2023), also showed upregulation, aligning with Etravirine's synaptogenic profile. PAX2, a transcription factor crucial on neurodevelopment whose mutations are associated with intellectual disability, epilepsy, and ASD (Serra *et al.,* 2022), was similarly upregulated, suggesting restoration of neurodevelopmental transcriptional control. Finally, CHN1 (α2-chimaerin), reported to harbor variants associated with ASD in a high-impact genetic study (Bacchelli *et al.,* 2003), was downregulated, potentially reflecting normalization of aberrant signaling pathways involved in axon pruning and neuronal connectivity.

Collectively, these transcriptomic changes support a convergent mechanism whereby Etravirine modulates key genes and kinases involved in synaptic architecture, neuronal maturation, and neurodevelopmental homeostasis. Interestingly, the enriched molecular pathways associated with the restoration of the FMR1 autistic phenotype underscore the strong relevance of Etravirine to core behavioral deficits in autism, including cognition, sensory processing, and social interaction (Fig. 4A). Furthermore, this analysis revealed that the altered genes are implicated in several pathways in brain development-particularly those involving neuronal connectivity, synaptic formation and neuronal plasticity. Finally, the dysregulated genes in FMR1 mice brains following Etravirine treatment are involved in molecular functions frequently disrupted in altered homeostasis, such as tissue remodeling, inflammation (suggesting neuroinflammatory activity), and kinase regulation, further supporting Etravirine's impact on a broad range of kinases (Fig. 4A). These findings provide a mechanistic rationale for the composition's application in ASD and related disorders characterized by dysregulated kinase activity and aberrant gene expression.

The inventors have also performed an experiment assessing β-catenin fluorescence activity in response to Etravirine treatment, illustrating its effects on Wnt signaling activity (Figure 3). The findings indicate that Etravirine has the ability to activate the Wnt pathway, comparable to the effects of a known Wnt activator. This suggests that Etravirine may restore Wnt pathway function in neurodevelopmental disorders where its activity is dysregulated. Transcriptomic analysis of brain tissue from FMR1-knockout (autistic) mice treated with Etravirine further confirms the activation of Wnt signaling (Fig. 4B) and Wnt-regulated genes (Fig. 4C) , along with other autism-associated pathways (Fig. 4A).

The canonical Wnt signaling pathway is strongly connected with numerous molecular and cellular processes, influencing development, tissue homeostasis, and disease (Liu *et al.,* 2022). Specifically, the canonical Wnt pathway regulates key gene transcription by translocating stabilized β-catenin to the nucleus and activating TCF/LEF transcription factors. Moreover, it is a key component of metabolic regulation, cell proliferation and survival, stem cell maintenance and embryonic development (Liu *et al.,* 2022).

Dysregulation of Wnt signaling has been shown to be significantly implicated in Autism Spectrum Disorders, as excessive Wnt-glycolysis signaling mediated by Axin-2 contributes to social deficits in ASD mouse models (M. Wang *et al.,* 2023; Bae & Hong, 2018) and its differentiated transcriptional activity contributes to the phenotypic heterogeneity in ASD (Caracci *et al.,* 2021).

Likewise, Wnt pathway is also noticeably related to ADHD disorder as studies have shown that stress-induced alterations in Wnt signaling enhance the ADHD pathogenesis, suggesting that modulating this pathway will offer novel therapeutic strategies (Yde Ohki *et al.,* 2020). Furthermore, proteomic analysis of the hippocampal dentate gyrus in a THRSP-overexpressing mouse model of ADHD indicate that alterations in Wnt signaling pathways are associated with behavioral impairments observed in this model, suggesting a critical role for Wnt signaling in ADHD-related behaviors (Custodio *et al.,* 2023).

Collectively, these studies suggest that dysregulation of the canonical Wnt pathway is a critical factor underlying the pathophysiology of ASD and ADHD, contributing to the occurrence of their characteristic phenotypic symptoms and associated neurodevelopmental impairments. The inventors provide strong indications that the composition possesses the ability to normalize and specifically activate the Wnt pathway, thereby restoring its functional integrity (Figure 3). Given the well-established role of Wnt signaling in neuronal development, synaptic plasticity, and cognitive processes, Etravirine's modulatory effects on this pathway hold significant therapeutic potential in mitigating the core deficits observed in ASD and ADHD. By targeting the dysregulated Wnt signaling cascade, the composition facilitates the restoration of neurodevelopmental processes that are otherwise impaired in these disorders, ultimately contributing to the development of an effective therapeutic intervention for individuals affected by ASD and ADHD. Thus, patients exhibiting dysregulation of the canonical Wnt signaling pathway-commonly implicated in ASD and ADHD-benefit from administration of the pharmaceutical composition of the present invention, which targets this pathway to restore key neurodevelopmental processes.

To confirm their hypothesis, the inventors performed *in vivo* experiments on one of the most robust models of ASD (BTBR mouse) and of ADHD (DAT-KO mouse). The behavioural experiments demonstrated that Etravirine unexpectedly ameliorated the ASD and ADHD-related phenotypes in BTBR and DAT-KO mice, respectively. Based on the interpretation of these findings, Etravirine emerges as a promising candidate for the treatment of neurodevelopmental disorders, including Autism and ADHD. More specifically, upon Etravirine treatment, social deficits in both BTBR and DAT-KO mice were ameliorated, reaching healthy wild-type mic levels (WT). Hyperactivity and stereotypy in both models were also ameliorated upon Etravirine administration; Etravirine was able to restore compulsivity, cognitive impairment, impaired memory and learning, attention deficit, and cognitive functions almost to wild-type levels.

The composition is intended for the treatment of neurodevelopmental diseases and disorders associated with the significant dysregulation of the canonical Wnt pathway that can be normalised by Etravirine. Disorders associated with disruptions in this specific pathway include 22q11.2 Deletion Syndrome (DiGeorge Syndrome), Cornelia de Lange Syndrome (CdLS), Prader-Willi Syndrome and Obsessive-Compulsive Disorder (OCD). 22q11.2 Deletion Syndrome (DiGeorge Syndrome) is characterised as a genetic disorder caused by a microdeletion on chromosome 22q11.2, characterized by congenital heart defects, immune dysfunction, developmental delays, and increased risk for neuropsychiatric conditions such as schizophrenia and ADHD. Recent studies in mice have proven that dysregulated expression of the canonical beta catenin- Wnt pathway strongly induces DiGeorge syndrome phenotypes (Huh & Ornitz, 2010). Cornelia de Lange Syndrome (CdLS) is a rare multisystem developmental disorder resulting from mutations in genes regulating cohesin function, marked by distinctive facial features, growth retardation, limb abnormalities, intellectual disability, and behavioral challenges including autistic traits. Mutations in cohesin complex genes (NIPBL, SMC1A) disrupt Wnt signaling in Cornelia de Lange patients which is critical for CNS development (Avagliano *et al.,* 2017). Prader-Willi Syndrome exhibits a complex genetic syndrome, characterized by hypotonia, hyperphagia leading to obesity, intellectual disability, and behavioral problems such as obsessive tendencies and mood dysregulation. Studies have shown that the Peg12/Frat3 region in mice genome which has syntenic homology to the human Prader-Willi syndrome region, is over-expressed in pathological conditions. Peg12/Frat3 is expressed primarily in embryonic stages and might be a positive regulator of the Wnt signaling pathway, suggesting a significant connection between disrupted expression of Wnt pathway and Prader-Willi Syndrome phenotype (Kobayashi *et al.,* 2002). Finally, lithium which is an established GSK3β inhibitor, regulating the physiological expression of the Wnt pathway, is suggested as a therapeutic component in Obsessive-Compulsive Disorder (OCD), supporting that its dysregulation importantly influences OCD observable traits (Vallee *et al.,* 2021).

The composition may be utilized for the treatment of diseases significantly impacted by the dysregulation Wnt pathway analyzed above.

### Examples

### Example Formulations and Treatments

A number of example formulations are provided below along with suggested dosage regimes. It should be understood that these are for illustrative purposes and these would be optimised during further experimentation, which may include clinical trials. For clarity, non-active components -such as pharmaceutically acceptable carriers or excipients- are not specified in these formulations

### Formulation 2A - Etravirine - Oral Tablet for the Treatment Autism and ADHD

| **Active Ingredient** | **Form** | **mg** | **Dose** |
|---|---|---|---|
| Etravirine | Oral Tablet | 100 - 800 | total daily |

The skilled addressee should understand that therapeutically effective doses depend on the activity and formulation of the chosen pharmaceutically active ingredient.

The foregoing embodiments are not intended to limit the scope of the protection afforded by the claims, but rather to describe examples of how the invention may be put into practice.

### Bibliography

Allavena, C., Katlama, C., Cotte, L., Roger, P. M., Delobel, P., Cheret, A., Dat, A. S. g. (2016). Long-term efficacy and safety of etravirine-containing regimens in a real-life cohort of treatment-experienced HIV-1-infected patients. Infect Dis (Lond), 48(5), 392-398. doi: 10.3109/23744235.2015.1133927
Amodeo, D. A., Rivera, E., Dunn, J. T., & Ragozzino, M. E. (2016). M100907 attenuates elevated grooming behavior in the BTBR mouse. Behavioural Brain Research, 313, 67-70. https://doi.org/10.1016/J.BBR.2016.06.064
Amodeo, D. A., Yi, J., Sweeney, J. A., & Ragozzino, M. E. (2014). Oxotremorine treatment reduces repetitive behaviors in BTBR T+ tf/J mice. Frontiers in Synaptic Neuroscience, 6(JUL). https://doi.org/10.3389/FNSYN.2014.00017
Avagliano, L., Grazioli, P., Mariani, M., Bulfamante, G. P., Selicorni, A., & Massa, V. (2017). Integrating molecular and structural findings: Wnt as a possible actor in shaping cognitive impairment in Cornelia de Lange syndrome. Orphanet J Rare Dis, 12(1), 174. doi: 10.1186/s13023-017-0723-0
Bacchelli, E., Blasi, F., Biondolillo, M., Lamb, J. A., Bonora, E., Barnby, G., . . . International Molecular Genetic Study of Autism, C. (2003). Screening of nine candidate genes for autism on chromosome 2q reveals rare nonsynonymous variants in the cAMP-GEFII gene. Mol Psychiatry, 8(11), 916-924. doi: 10.1038/sj.mp.4001340
Bae, S. M., & Hong, J. Y. (2018). The Wnt Signaling Pathway and Related Therapeutic Drugs in Autism Spectrum Disorder. Clin Psychopharmacol Neurosci, 16(2), 129-135. doi: 10.9758/cpn.2018.16.2.129
Bakheet, S. A., Alzahrani, M. Z., Nadeem, A., Ansari, M. A., Zoheir, K. M. A., Attia, S. M., AL-Ayadhi, L. Y., & Ahmad, S. F. (2016). Resveratrol treatment attenuates chemokine receptor expression in the BTBR T+tf/J mouse model of autism. Molecular and Cellular Neurosciences, 77, 1-10. https://doi.org/10.1016/J.MCN.2016.09.004
Balogh, L., Pulay, A. J., & Réthelyi, J. M. (2022). Genetics in the ADHD Clinic: How Can Genetic Testing Support the Current Clinical Practice? Frontiers in Psychology, 13, 751041. https://doi.org/10.3389/FPSYG.2022.751041
Blum, K., Chen, A. L. C., Braverman, E. R., Comings, D. E., Chen, T. J. H., Arcuri, V., Blum, S. H., Downs, B. W., Waite, R. L., Notaro, A., Lubar, J., Williams, L., Prihoda, T. J., Palomo, T., & Oscar-Berman, M. (2008). Attention-deficit-hyperactivity disorder and reward deficiency syndrome. Neuropsychiatric Disease and Treatment, 4(5), 893. https://doi.org/10.2147/NDT.S2627
Bossé, R., Fumagalli, F., Jaber, M., Giros, B., Gainetdinov, R. R., Wetsel, W. C., Missale, C., & Caron, M. G. (1997). Anterior pituitary hypoplasia and dwarfism in mice lacking the dopamine transporter. Neuron, 19(1), 127-138. https://doi.org/10.1016/S0896-6273(00)80353-0
Brown, A. B., Biederman, J., Valera, E. M., Doyle, A. E., Bush, G., Spencer, T., Monuteaux, M. C., Mick, E., Whitfield-Gabrieli, S., Makris, N., LaViolette, P. S., Oscar-Berman, M., Faraone, S. V., & Seidman, L. J. (2010). Effect of Dopamine Transporter Gene (SLC6A3) Variation on Dorsal Anterior Cingulate Function in Attention-Deficit/Hyperactivity Disorder. American Journal of Medical Genetics. Part B, Neuropsychiatric Genetics : The Official Publication of the International Society of Psychiatric Genetics, 153B(2), 365. https://doi.org/10.1002/AJMG.B.31022
Burket, J. A., Benson, A. D., Tang, A. H., & Deutsch, S. I. (2014). Rapamycin improves sociability in the BTBR T(+)Itpr3(tf)/J mouse model of autism spectrum disorders. Brain Research Bulletin, 100, 70-75. https://doi.org/10.1016/J.BRAINRESBULL.2013.11.005
Burkhardt, J., Kirsten, H., Holland, H., Krupp, W., Ligges, C., Quente, E., Wilcke, A. (2012). Association of rs2069459 in the CDK5 gene with dyslexia in a German cohort. Psychiatr Genet, 22(6), 307-308. doi: 10.1097/YPG.0b013e328353aeae
Bustos, F., Segarra-Fas, A., Nardocci, G., Cassidy, A., Antico, O., Davidson, L., Findlay, G. M. (2020). Functional Diversification of SRSF Protein Kinase to Control Ubiquitin-Dependent Neurodevelopmental Signaling. Dev Cell, 55(5), 629-647 e627. doi: 10.1016/j.devcel.2020.09.025
Byeon, S., & Yadav, S. (2024). Pleiotropic functions of TAO kinases and their dysregulation in neurological disorders. Sci Signal, 17(817), eadg0876. doi: 10.1126/scisignal.adg0876
Caracci, M. O., Avila, M. E., Espinoza-Cavieres, F. A., Lopez, H. R., Ugarte, G. D., & De Ferrari, G. V. (2021). Wnt/beta-Catenin-Dependent Transcription in Autism Spectrum Disorders. Front Mol Neurosci, 14, 764756. doi: 10.3389/fnmol.2021.764756
Custodio, R. J. P., Kim, H. J., Kim, J., Ortiz, D. M., Kim, M., Buctot, D., Cheong, J. H. (2023). Hippocampal dentate gyri proteomics reveals Wnt signaling involvement in the behavioral impairment in the THRSP-overexpressing ADHD mouse model. Commun Biol, 6(1), 55. doi: 10.1038/s42003-022-04387-5
Cyr, M., Beaulieu, J. M., Laakso, A., Sotnikova, T. D., Yao, W. D., Bohn, L. M., Gainetdinov, R. R., & Caron, M. G. (2003). Sustained elevation of extracellular dopamine causes motor dysfunction and selective degeneration of striatal GABAergic neurons. Proceedings of the National Academy of Sciences of the United States of America, 100(19), 11035-11040. https://doi.org/10.1073/PNAS.1831768100
Davico, C., Secci, I., Vendrametto, V., & Vitiello, B. (2023). Pharmacological treatments in autism spectrum disorder: a narrative review. Journal of Psychopathology, 29(1-2), 38-52. https://doi.org/10.36148/2284-0249-N251
De Groot, T., Damen, L., Kosse, L., Alsady, M., Doty, R., Baumgarten, R., Sheehan, S., Van Der Vlag, J., Korstanje, R., & Deen, P. M. T. (2017). Lithium reduces blood glucose levels, but aggravates albuminuria in BTBR-ob/ob mice. PloS One, 12(12). https://doi.org/10.1371/JOURNALPONE.0189485
Del'Guidice, T., Lemasson, M., Etiévant, A., Manta, S., Magno, L. A. V., Escoffier, G., Roman, F. S., & Beaulieu, J. M. (2014). Dissociations between cognitive and motor effects of psychostimulants and atomoxetine in hyperactive DAT-KO mice. Psychopharmacology, 231(1), 109-122. https://doi.org/10.1007/S00213-013-3212-8
Drechsler, R., Brem, S., Brandeis, D., Grünblatt, E., Berger, G., & Walitza, S. (2020). ADHD: Current Concepts and Treatments in Children and Adolescents. Neuropediatrics, 51(5), 315. https://doi.org/10.1055/S-0040-1701658
Efimova, E. V., Gainetdinov, R. R., Budygin, E. A., & Sotnikova, T. D. (2016). Dopamine transporter mutant animals: a translational perspective. Journal of Neurogenetics, 30(1), 5. https://doi.org/10.3109/01677063.2016.1144751
Egger, J. I. M., Verhoeven, W. M. A., Groenendijk-Reijenga, R., & Kant, S. G. (2017). Phelan-McDermid syndrome due to SHANK3 mutation in an intellectually disabled adult male: successful treatment with lithium. BMJ Case Rep, 2017. doi: 10.1136/bcr-2017-220778
Endo, N., Hiraishi, A., Goto, S., Nozu, H., Mannari-Sasagawa, T., Horii-Hayashi, N., Kitsuki, M., Okuda, M., Makinodan, M., & Nishi, M. (2025). Dysregulated HPA axis during postnatal developmental stages in the BTBR T+ Itpr3tf/J mouse: A model of autism spectrum disorder. Neuropsychopharmacology Reports, 45(1), e12508. https://doi.org/10.1002/NPR2.12508
Engmann, O., Hortobagyi, T., Pidsley, R., Troakes, C., Bernstein, H. G., Kreutz, M. R., . . . Giese, K. P. (2011). Schizophrenia is associated with dysregulation of a Cdk5 activator that regulates synaptic protein expression and cognition. Brain, 134(Pt 8), 2408-2421. doi: 10.1093/brain/awr155
Fu, M., Hu, Y., Lan, T., Guan, K. L., Luo, T., & Luo, M. (2022). The Hippo signalling pathway and its implications in human health and diseases. Signal Transduct Target Ther, 7(1), 376. doi: 10.1038/s41392-022-01191-9
Gassowska, M., Baranowska-Bosiacka, I., Moczydlowska, J., Tarnowski, M., Pilutin, A., Gutowska, I., . . . Adamczyk, A. (2016). Perinatal exposure to lead (Pb) promotes Tau phosphorylation in the rat brain in a GSK-3beta and CDK5 dependent manner: Relevance to neurological disorders. Toxicology, 347-349, 17-28. doi: 10.1016/j.tox.2016.03.002
Gilbert, J., & Man, H.-Y. (2016). The X-Linked Autism Protein KIAA2022/KIDLIA Regulates Neurite Outgrowth via N-Cadherin and δ-Catenin Signaling. Eneuro, 3(5), ENEURO.0238-16.2016. https://doi.org/10.1523/ENEURO.0238-16.2016
Gilbert, J., & Man, H.-Y. (2017). Fundamental Elements in Autism: From Neurogenesis and Neurite Growth to Synaptic Plasticity. Frontiers in Cellular Neuroscience, 11. https://doi.org/10.3389/fncel.2017.00359
Gory-Faure, S., Powell, R., Jonckheere, J., Lante, F., Denarier, E., Peris, L.,Andrieux, A. (2021). Pyr1-Mediated Pharmacological Inhibition of LIM Kinase Restores Synaptic Plasticity and Normal Behavior in a Mouse Model of Schizophrenia. Front Pharmacol, 12, 627995. doi: 10.3389/fphar.2021.627995
Hall, F. S., Sora, I., Hen, R., & Uhl, G. R. (2014). Serotonin/Dopamine Interactions in a Hyperactive Mouse: Reduced Serotonin Receptor 1B Activity Reverses Effects of Dopamine Transporter Knockout. PLOS ONE, 9(12), e115009. https://doi.org/10.1371/JOURNAL.PONE.0115009
Hours, C., Recasens, C., & Baleyte, J.-M. (2022). ASD and ADHD Comorbidity: What Are We Talking About? Frontiers in Psychiatry, 13, 837424. https://doi.org/10.3389/FPSYT.2022.837424
Huh, S. H., & Ornitz, D. M. (2010). Beta-catenin deficiency causes DiGeorge syndrome-like phenotypes through regulation of Tbx1. Development, 137(7), 1137-1147. doi: 10.1242/dev.045534
Jiang, C. C., Lin, L. S., Long, S., Ke, X. Y., Fukunaga, K., Lu, Y. M., & Han, F. (2022). Signalling pathways in autism spectrum disorder: mechanisms and therapeutic implications. Signal Transduct Target Ther, 7(1), 229. doi: 10.1038/s41392-022-01081-0
Jones, T., Raman, R., Puhl, A. C., Lane, T. R., Riabova, O., Kazakova, E., Ekins, S. (2025). Discovery of Dual Targeting GSK-3beta/HIV-1 Reverse Transcriptase Inhibitors as Neuroprotective Antiviral Agents. ACS Chem Neurosci, 16(1), 77-84. doi: 10.1021/acschemneuro.4c00725
Jorge-Torres, O. C., Szczesna, K., Roa, L., Casal, C., Gonzalez-Somermeyer, L., Soler, M., Esteller, M. (2018). Inhibition of Gsk3b Reduces Nfkb1 Signaling and Rescues Synaptic Activity to Improve the Rett Syndrome Phenotype in Mecp2-Knockout Mice. Cell Rep, 23(6), 1665-1677. doi: 10.1016/j.celrep.2018.04.010
Kim, Y., Jeon, S., Jeong, H. J., Lee, S. M., Pena, I. D., Kim, H. J.,Cheong, J. H. (2019). Restoration of Cdk5, TrkB and Soluble N-ethylmaleimide-Sensitive Factor Attachment Protein Receptor Proteins after Chronic Methylphenidate Treatment in Spontaneous Hypertensive Rats, a Model for Attention-Deficit Hyperactivity Disorder. Psychiatry Investig, 16(7), 558-564. doi: 10.30773/pi.2019.04.22
Kobayashi, S., Kohda, T., Ichikawa, H., Ogura, A., Ohki, M., Kaneko-Ishino, T., & Ishino, F. (2002). Paternal expression of a novel imprinted gene, Peg12/Frat3, in the mouse 7C region homologous to the Prader-Willi syndrome region. Biochem Biophys Res Commun, 290(1), 403-408. doi: 10.1006/bbrc.2001.6160
LeClerc, S., & Easley, D. (2015). Pharmacological Therapies for Autism Spectrum Disorder: A Review. Pharmacy and Therapeutics, 40(6), 389. https://pmc.ncbi.nlm.nih.gov/articles/PMC4450669/
Li, Q., Shi, Y., Li, X., Yang, Y., Zhang, X., Xu, L., Ma, Z., Wang, J., Fan, L., & Wu, L. (2022). Proteomic-Based Approach Reveals the Involvement of Apolipoprotein A-I in Related Phenotypes of Autism Spectrum Disorder in the BTBR Mouse Model. International Journal of Molecular Sciences, 23(23). https://doi.org/10.3390/IJMS232315290
Liang, Z., Ye, T., Zhou, X., Lai, K. O., Fu, A. K., & Ip, N. Y. (2015). Cdk5 Regulates Activity-Dependent Gene Expression and Dendrite Development. J Neurosci, 35(45), 15127-15134. doi: 10.1523/JNEUROSCI.1443-15.2015
Liu, J., Xiao, Q., Xiao, J., Niu, C., Li, Y., Zhang, X., Yin, G. (2022). Wnt/β-catenin signalling: function, biological mechanisms, and therapeutic opportunities. Signal Transduct Target Ther, 7(1), 3. doi: 10.1038/s41392-021-00762-6
Long, I. (2024). Editorial: Common pathways in neurodevelopmental conditions and neurodegenerative disorders. Front Neurosci, 18, 1454384. doi: 10.3389/fnins.2024.1454384
Lord, C., Elsabbagh, M., Baird, G., & Veenstra-Vanderweele, J. (2018). Autism spectrum disorder. Lancet (London, England), 392(10146), 508. https://doi.org/10.1016/S0140-6736(18)31129-2
Mak, B. C., Kenerson, H. L., Aicher, L. D., Barnes, E. A., & Yeung, R. S. (2005). Aberrant beta-catenin signaling in tuberous sclerosis. Am J Pathol, 167(1), 107-116. doi: 10.1016/s0002-9440(10)62958-6
Matrone, C., & Ferretti, G. (2023). Semaphorin 3A influences neuronal processes that are altered in patients with autism spectrum disorder: Potential diagnostic and therapeutic implications. Neurosci Biobehav Rev, 153, 105338. doi: 10.1016/j.neubiorev.2023.105338
McFarlane, H. G., Kusek, G. K., Yang, M., Phoenix, J. L., Bolivar, V. J., & Crawley, J. N. (2008). Autism-like behavioral phenotypes in BTBR T+tf/J mice. Genes, Brain, and Behavior, 7(2), 152-163. https://doi.org/10.1111/J.1601-183X.2007.00330.X
Mechler, K., Banaschewski, T., Hohmann, S., & Häge, A. (2022). Evidence-based pharmacological treatment options for ADHD in children and adolescents. Pharmacology & Therapeutics, 230, 107940. https://doi.org/10.1016/J.PHARMTHERA.2021.107940
Meyza, K. Z., & Blanchard, D. C. (2017). The BTBR mouse model of idiopathic autism - Current view on mechanisms. Neuroscience and Biobehavioral Reviews, 76(Pt A), 99-110. https://doi.org/10.1016/J.NEUBIOREV.2016.12.037
Mines, M. A., Yuskaitis, C. J., King, M. K., Beurel, E., & Jope, R. S. (2010). GSK3 influences social preference and anxiety-related behaviors during social interaction in a mouse model of fragile X syndrome and autism. PLoS One, 5(3), e9706. doi: 10.1371/journal.pone.0009706
Moncini, S., Castronovo, P., Murgia, A., Russo, S., Bedeschi, M. F., Lunghi, M., Venturin, M. (2016). Functional characterization of CDK5 and CDK5R1 mutations identified in patients with non-syndromic intellectual disability. J Hum Genet, 61(4), 283-293. doi: 10.1038/jhg.2015.144
Mutovina, A., Ayriyants, K., Mezhlumyan, E., Ryabushkina, Y., Litvinova, E., Bondar, N., Khantakova, J., & Reshetnikov, V. (2022). Unique Features of the Immune Response in BTBR Mice. International Journal of Molecular Sciences, 23(24). https://doi.org/10.3390/IJMS232415577
Nisar, S., Hashem, S., Bhat, A. A., Syed, N., Yadav, S., Azeem, M. W., Uddin, S., Bagga, P., Reddy, R., & Haris, M. (2019). Association of genes with phenotype in autism spectrum disorder. Aging (Albany NY), 11(22), 10742. https://doi.org/10.18632/AGING.102473
Pande, C. K., O'Halloran, A., Stewart, R. W., Nguyen, A., & Canares, T. (2019). Case 2: Agitation and Abnormal Movements in a 14-year-old Boy. Pediatr Rev, 40(10), 532-534. doi: 10.1542/pir.2017-0221
Pinto, D., Pagnamenta, A. T., Klei, L., Anney, R., Merico, D., Regan, R., Conroy, J., Magalhaes, T. R., Correia, C., Abrahams, B. S., Almeida, J., Bacchelli, E., Bader, G. D., Bailey, A. J., Baird, G., Battaglia, A., Berney, T., Bolshakova, N., Bölte, S. Betancur, C. (2010). Functional impact of global rare copy number variation in autism spectrum disorders. Nature, 466(7304), 368-372. https://doi.org/10.1038/nature09146
Polanczyk, G., De Lima, M. S., Horta, B. L., Biederman, J., & Rohde, L. A. (2007). The worldwide prevalence of ADHD: a systematic review and metaregression analysis. The American Journal of Psychiatry, 164(6), 942-948.
Ramirez-Celis, A., Croen, L. A., Yoshida, C. K., Alexeeff, S. E., Schauer, J., Yolken, R. H.,Van de Water, J. (2022). Maternal autoantibody profiles as biomarkers for ASD and ASD with co-occurring intellectual disability. Mol Psychiatry, 27(9), 3760-3767. doi: 10.1038/s41380-022-01633-4
Scholler-Gyure, M., Kakuda, T. N., Raoof, A., De Smedt, G., & Hoetelmans, R. M. (2009). Clinical pharmacokinetics and pharmacodynamics of etravirine. Clin Pharmacokinet, 48(9), 561-574. doi: 10.2165/10895940-000000000-00000
Serra, I., Stravs, A., Osorio, C., Oyaga, M. R., Schonewille, M., Tudorache, C., & Badura, A. (2022). Tsc1 Haploinsufficiency Leads to Pax2 Dysregulation in the Developing Murine Cerebellum. Front Mol Neurosci, 15, 831687. doi: 10.3389/fnmol.2022.831687
Stamova, B. S., Tian, Y., Nordahl, C. W., Shen, M. D., Rogers, S., Amaral, D. G., & Sharp, F. R. (2013). Evidence for differential alternative splicing in blood of young boys with autism spectrum disorders. Mol Autism, 4(1), 30. doi: 10.1186/2040-2392-4-30
Tsutiya, A., Nakano, Y., Hansen-Kiss, E., Kelly, B., Nishihara, M., Goshima, Y., Ohtani-Kaneko, R. (2017). Human CRMP4 mutation and disrupted Crmp4 expression in mice are associated with ASD characteristics and sexual dimorphism. Sci Rep, 7(1), 16812. doi: 10.1038/s41598-017-16782-8
Vallee, A., Vallee, J. N., & Lecarpentier, Y. (2021). Lithium: a potential therapeutic strategy in obsessive-compulsive disorder by targeting the canonical WNT/beta pathway. Transl Psychiatry, 11(1), 204. doi: 10.1038/s41398-021-01329-3
Varghese, M., Keshav, N., Jacot-Descombes, S., Warda, T., Wicinski, B., Dickstein, D. L., Harony-Nicolas, H., De Rubeis, S., Drapeau, E., Buxbaum, J. D., & Hof, P. R. (2017). Autism spectrum disorder: neuropathology and animal models. Acta Neuropathologica, 134(4), 537-566. https://doi.org/10.1007/s00401-017-1736-4
Viggiano, D., Ruocco, L. A., & Sadile, A. G. (2003). Dopamine phenotype and behaviour in animal models: In relation to attention deficit hyperactivity disorder. Neuroscience and Biobehavioral Reviews, 27(7), 623-637. https://doi.org/10.1016/j.neubiorev.2003.08.006
Viscomi, M. P., Czyrska, J., Winiarczyk, D., Zi 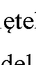 tek, M. M., & Sampino, S. (2025). The BTBR T+ Itpr3tf/J mouse strain as a model to study the genetic, immune, and metabolic origins of neurodevelopmental disorders. Research in Autism Spectrum Disorders, 119, 102526. https://doi.org/10.1016/J.RASD.2024.102526
Wang, J., Li, W., Li, Z., Xue, Z., Zhang, Y., Yuan, Y., Qiu, Z. (2023). Taok1 haploinsufficiency leads to autistic-like behaviors in mice via the dorsal raphe nucleus. Cell Rep, 42(9), 113078. doi: 10.1016/j.celrep.2023.113078
Wang, J. J., Roy, S. K., & Xu, Y. M. (2024). Spatiotemporal expression and coexpression patterns of SRPK1 in the human brain: A neurodevelopmental perspective. Brain Behav, 14(1), e3341. doi: 10.1002/brb3.3341
Wang, M., Xian, P., Zheng, W., Li, Z., Chen, A., Xiao, H.,Wu, S. (2023). Axin2 coupled excessive Wnt-glycolysis signaling mediates social defect in autism spectrum disorders. EMBO Mol Med, 15(6), e17101. doi: 10.15252/emmm.202217101
Xia, D., Zhang, L., Mei, R., Wu, C., Liu, Y., Chen, H., & Chen, L. (2025). Increased Expression of MST1 in Patients With Epilepsy and in a Rat Model of Epilepsy. Synapse, 79(1), e70002. doi: 10.1002/syn.70002
Yang, Y., Wang, L., Wu, Y., Su, D., Wang, N., Wang, J., . . . Zhang, S. (2016). Tanshinol suppresses inflammatory factors in a rat model of vascular dementia and protects LPS-treated neurons via the MST1-FOXO3 signaling pathway. Brain Res, 1646, 304-314. doi: 10.1016/j.brainres.2016.06.017
Yao, F., Zhang, K., Feng, C., Gao, Y., Shen, L., Liu, X., & Ni, J. (2021). Protein Biomarkers of Autism Spectrum Disorder Identified by Computational and Experimental Methods. Front Psychiatry, 12, 554621. doi: 10.3389/fpsyt.2021.554621
Yde Ohki, C. M., Grossmann, L., Alber, E., Dwivedi, T., Berger, G., Werling, A. M., .Grunblatt, E. (2020). The stress-Wnt-signaling axis: a hypothesis for attention-deficit hyperactivity disorder and therapy approaches. Transl Psychiatry, 10(1), 315. doi: 10.1038/s41398-020-00999-9
Yen, Y. C., Gassen, N. C., Zellner, A., Rein, T., Landgraf, R., Wotjak, C. T., & Anderzhanova, E. (2015). Glycogen synthase kinase-3beta inhibition in the medial prefrontal cortex mediates paradoxical amphetamine action in a mouse model of ADHD. Front Behav Neurosci, 9, 67. doi: 10.3389/fnbeh.2015.00067
Zerbi, V., Pagani, M., Markicevic, M., Matteoli, M., Pozzi, D., Fagiolini, M., Bozzi, Y., Galbusera, A., Scattoni, M. L., Provenzano, G., Banerjee, A., Helmchen, F., Basson, M. A., Ellegood, J., Lerch, J. P., Rudin, M., Gozzi, A., & Wenderoth, N. (2021). Brain mapping across 16 autism mouse models reveals a spectrum of functional connectivity subtypes. Molecular Psychiatry 2021 26:12, 26(12), 7610-7620. https://doi.org/10.1038/s41380-021-01245-4
Zhang, Z., Zhang, P., Qi, G. J., Jiao, F. J., Wang, Q. Z., Yan, J. G.,Tian, B. (2018). CDK5-mediated phosphorylation of Sirt2 contributes to depressive-like behavior induced by social defeat stress. Biochim Biophys Acta Mol Basis Dis, 1864(2), 533-541. doi: 10.1016/j.bbadis.2017.11.012
Zhou, A., Cao, X., Mahaganapathy, V., Azaro, M., Gwin, C., Wilson, S., Buyske, S., Bartlett, C. W., Flax, J. F., Brzustowicz, L. M., & Xing, J. (2022). Common genetic risk factors in ASD and ADHD co-occurring families. Human Genetics, 142(2), 217. https://doi.org/10.1007/S00439-022-02496-Z
Zimmermann, A. M., Jene, T., Wolf, M., Gorlich, A., Gurniak, C. B., Sassoe-Pognetto, M., ... Rust, M. B. (2015). Attention-Deficit/Hyperactivity Disorder-like Phenotype in a Mouse Model with Impaired Actin Dynamics. Biol Psychiatry, 78(2), 95-106. doi: 10.1016/j.biopsych.2014.03.011

## Claims

1. Etravirine for use in the treatment of a neurodevelopmental disorder.

2. Etravirine for the use as claimed in claim 1, wherein the neurodevelopmental disorder is associated with kinase dysregulation impacting synaptic plasticity, neuronal development, or cognitive function.

3. Etravirine for the use as claimed in claim 1 or 2, wherein the neurodevelopmental disorder is Autism Spectrum Disorder (ASD).

4. Etravirine for the use as claimed in claim 1 or 2, wherein the neurodevelopmental disorder is Attention-Deficit/Hyperactivity Disorder (ADHD).

5. Etravirine for the use as claimed in any one of claims 1 to 4, wherein the subject to be treated exhibits a molecular dysregulation in a pathway mediated by one or more of the following kinases: Cyclin-Dependent Kinase 5 (CDK5), LIM Kinase 1 (LIMK1), Serine/threonine-protein kinase TAO1 (TAOK1), Serine/Arginine-Rich Protein-Specific Kinase 1 (SRPK1), Glycogen Synthase Kinase 3 Beta (GSK3β), Mammalian Ste20-like Kinase 1 (MST1), and Casein Kinase 1 Gamma 1 (CK1γ1).

6. Etravirine for the use as claimed in any one of claims 1 to 4, wherein the subject to be treated exhibits a dysregulation of the canonical Wnt signaling pathway.

7. A pharmaceutical composition comprising Etravirine and a pharmaceutical excipient for use in the treatment of a neurodevelopmental disorder.

8. The pharmaceutical composition for use as claimed in claim 7, wherein the pharmaceutical excipient is selected from diluents, binders, disintegrants, glidants and lubricants.

9. The pharmaceutical composition for use as claimed in claim 7 or 8, wherein the pharmaceutical composition is a tablet.

10. The pharmaceutical composition for use as claimed in any one of claims 7 to 9, wherein the pharmaceutical composition contains 25 mg to 200 mg, preferably 25 mg, 100 mg or 200 mg Etravirine.

11. The pharmaceutical composition for use as claimed in any one of claims 7 to 10, wherein Etravirine is to be administered in a daily dose of 100 mg to 800 mg, preferably 200 mg to 400 mg.

12. The pharmaceutical composition for use as claimed in any one of claims 7 to 11, wherein the pharmaceutical composition is to be administered separately, concurrently, or sequentially with another pharmaceutically active ingredient.

13. The pharmaceutical composition for use as claimed in claim 12, wherein the other pharmaceutically active ingredient is a selective serotonin reuptake inhibitor (SSRI).

14. The pharmaceutical composition for use as claimed in any one of claims 7 to 13, wherein the neurodevelopmental disorder is selected from ASD, ADHD, Fragile X Syndrome (FXS), Rett Syndrome, Angelman Syndrome, Phelan-McDermid Syndrome, Tuberous Sclerosis Complex (TSC), Schizophrenia, Bipolar Disorder, Major Depressive Disorder (MDD), Obsessive-Compulsive Disorder (OCD), Intellectual Disability (ID), epilepsy-related cognitive deficits, DiGeorge Syndrome, Cornelia de Lange Syndrome (CdLS), and Prader-Willi Syndrome.
